# EUROPEAN PATENT APPLICATION

(11) **EP 0 639 366 A1**
(43) Date of publication of application: **22.02.1995**
(21) Application number: 93306559.1
(22) Date of filing: 19.08.1993
(51) Int. Cl.: A61K 6/033, A61L 27/00

(54) **Hydroxyapatite cement as bone or tooth replacement**

(71) Applicant: KINGSTAR TECHNOLOGY LIMITED (UK), London W1Y 9DD (GB)
(72) Inventor: Li, Chen, Shanghai (CN)
(74) Representative: Luckhurst, Anthony Henry William

(57) **Abstract**

In order to produce a hydroxyapatite cement useful in bone and tooth replacement, a calcium salt and a calcium phosphate are admixed in an aqueous solution of base, phosphate or organic acid capable of forming calcium ligand complexes, to deposit a hydroxyapatite cement, and/or the ratio of the total molar quantity of calcium to phosphate is in the range 1.3 : 1 to 1.8 : 1.

## Description

The present invention relates to a process and to preparations for use in the production of hydroxyapatite cements which are useful in bone and tooth replacement.

Hydroxyapatite [Ca₅(PO₄)₃OH] is a calcium phosphate compound and is the major mineral component of bones and teeth, and can be used to fill cavities in teeth, or as a bone implant. Hydroxyapatite can exist in a number of forms such as a powder or ceramic, but the cement is the most important in bone and tooth replacement.

Hydroxyapatite cement is preferable to currently available biomaterials such as β-tricalcium phosphate, bioglass, biphasic calcium phosphate and polymethyl methacrylate (PMMA), because, in contrast to such materials, (1) it is highly biocompatible, (2) it sets to a hard mass with strength sufficient for many medical and dental applications, and (3) when implanted in bone, it resorbs and is eventually replaced by new bone tissue with no loss in volume or integrity.

US-A-5 053 212 discloses a process for producing hydroxyapatite cement by reacting a powder comprising phosphoric acid crystals or monocalcium phosphate monohydrate with calcium carbonate, calcium oxide, calcium hydroxide or tetracalcium phosphate. However, the reactants have to be partially reacted during a dry milling process before water is added.

US Re33161 and US Re33221 disclose processes for the production cements of calcium phosphate, including hydroxyapatite cements, which comprise reacting a powder comprising tetracalcium phosphate and dicalcium phosphate dihydrate or anhydrous dicalcium phosphate with water or extremely dilute solution of acid, base, or salt (of the order of 20 mM). The cements formed have good compressive strength (of the order of 34MPa). These hydroxyapatite cements have good in vivo characteristics, in particular high biocompatibility and good osseointegration, but the setting time is of the order of 30 minutes, which is undesirably long.

The present inventors have discovered that, if a calcium salt and a salt containing calcium ions and phosphate ions are reacted together in the presence of water, complete conversion to hydroxyapatite cement can be achieved if the ratio of the total molar quantity of calcium to the total molar quantity of phosphate is substantially in the range 1.3 : 1 to 1.8 : 1. This ratio ensures virtually complete conversion to hydroxyapatite cement without the formation of side-products in sufficient quantities to be deleterious to the final product.

The present inventors have further discovered that a short setting time (for example, a setting time in the range of 5 to 15 minutes) can be achieved if a calcium salt and a salt containing calcium ions and phosphate ions are reacted in the presence of an aqueous solution of base or an aqueous solution of phosphate ions. Even shorter setting times, as short as one minute, can be achieved by using an aqueous solution of an organic acid capable of forming calcium ligand complexes, for example citric acid. Hydroxyapatite cements deposited from these solutions have greater strength than hydroxyapatite cements deposited from other liquids.

Thus, in a first aspect, the present invention provides a process for producing a hydroxyapatite cement for use in bone or tooth replacement, comprising admixing a first calcium salt and at least one further calcium salt containing phosphate ions in an aqueous environment to yield a hydroxyapatite cement, the ratio of the total molar quantity of calcium to the total molar quantity of phosphate being in the range 1.3 : 1 to 1.8 : 1.

In a second aspect, the present invention provides a process for producing a hydroxyapatite cement useful in bone or tooth replacement, comprising admixing a first calcium salt and at least one further calcium salt containing phosphate ions in a liquid so as to deposit a hydroxyapatite cement, characterised in that the liquid consists essentially of an aqueous solution of base, an aqueous solution of a phosphate and/or an aqueous solution of an organic acid capable of forming calcium ligand complexes.

It will be appreciated that the liquid may also comprise other ingredients as desired, but should essentially comprise a solution as defined, in order to provide the necessary reaction conditions.

Whilst it will be appreciated that the aqueous environment can comprise a mixture of two or more of the phosphate, base or organic acid, it is generally preferred that the solution should comprise only one of these. It is particularly preferred that the solution comprises organic acid, as this gives particularly short reaction times, a solution consisting essentially of organic acid being even more preferred.

The base, phosphate and/or organic acid may be present in a dilute solution, but the advantages of the invention will not usually be observed at concentrations below 0.1 M. We strongly prefer that the base, phosphate and/or organic acid are present in an appreciable concentration, in order to give a good reaction rate, so that a concentration of at least 0.2 M is preferred, most preferably at least 0.5 M. It will be appreciated that higher concentrations may be used and practicable concentrations will be apparent to the person skilled in the art. A total concentration of base, phosphate and/or organic acid less than or equal to 1.5 M is preferred, to avoid generating excess heat.

Where the liquid comprises an aqueous solution of base, then the base is preferably NaOH and/or KOH. If the liquid comprises a phosphate solution, the phosphate is preferably Na₃PO₄, Na₂HPO₄, NaH₂PO₄ and/or (NH₄)₂HPO₄. If the liquid comprises an organic acid, it is preferably citric acid and/or phthalic acid. All these substances are readily available and have good biocompatibility.

Where the hydroxyapatite cement is prepared by deposition from an aqueous solution of a base, phosphate and/or organic acid, then suitable calcium salts include calcium oxide (CaO), calcium hydroxide [Ca(OH)₂], calcium carbonate (CaCO₃), tetracalcium phosphate [Ca₄(PO₄)₂O], monocalcium phosphate monohydrate [Ca(H₂PO₄)₂.H₂O], anhydrous monocalcium phosphate (Ca(H₂PO₄)₂], dicalcium phosphate dihydrate (CaHPO₄.2H₂O), anhydrous dicalcium phosphate (CaHPO₄), octacalcium phosphate (Ca₈H₂ (PO₄)₆.5H₂O], amorphous calcium phosphate (Ca₃(PO₄)₂], α-tricalcium phosphate [α-Ca₃(PO₄)₂] and/or β-tricalcium phosphate [βCa₃(PO₄)₂].

In general, the first calcium salt and the further calcium salt are both preferably in the form of finely ground powder to increase the speed of the reaction. The average particle diameter is preferably below 50µm, particularly about or below 30µm. In some cases, an average particle diameter as small as 1µm is advantageous.

The material may be ground to a fine powder by using a compressed air jet pulveriser. If an average particle diameter of as low as 1µm is required, a high speed ball mill may be used. Ideally, the material should be ground in a water free, non-aqueous liquid so as to avoid reaction of the material with water.

It is preferred that the calcium salts are mixed with a minimum amount of aqueous liquid sufficient to yield hydroxyapatite cement with good mechanical strength. It is preferred that the amount of liquid added results in the formation of a thick paste.

Although it will be appreciated that a ratio in the range of 1.3 : 1 to 1.8 : 1 is advantageous, to avoid the formation of undesired side-products, the ratio of the total molar quantity of calcium to the total molar quantity of phosphate is more preferably in the range 1.6 : 1 to 1.75 : 1, a ratio of approximately 1.67 : 1 being most preferred, this being the ratio of calcium to phosphate in hydroxyapatite.

The first calcium salt is advantageously tetracalcium phosphate (Ca₄(PO₄)₂O], but may also be calcium oxide (CaO), calcium hydroxide [Ca(OH)₂], or calcium carbonate (CaCO₃). However, in these latter cases, the cement should be prepared in an aqueous solution of base, phosphate and/or organic acid in order to obtain useful cement.

In order to give a total calcium to phosphate ratio within the range 1.3 : 1 to 1.8 : 1, where desired, an appropriate quantity of further calcium salt is used, suitable further calcium salts being monocalcium phosphate monohydrate [Ca(H₂PO₄)₂.H₂O], anhydrous monocalcium phosphate [Ca(H₂PO₄)₂], dicalcium phosphate dihydrate (CaHPO₄.2H₂O), anhydrous dicalcium phosphate (CaHPO₄), octacalcium phosphate [Ca₈H₂ (PO₄)₆.5H₂O], amorphous calcium phosphate [Ca₃(PO₄)₂], α-tricalcium phosphate [α-Ca₃(PO₄)₂] and/or β-tricalcium phosphate [βCa₃(PO₄)₂].

It is preferred that the first calcium salt comprises calcium phosphate and that the further calcium salt is selected from the salts listed in Table I, the molar quantity of the further calcium salt per mole of tetracalcium phosphate required to give a total of calcium to phosphate in the preferred ratio of 1.67 : 1 being that which is specified in the right-hand column.

**TABLE I**

| Further calcium salt | Mols [per mole Ca₄(PO₄)₂O] |
|---|---|
| Ca(H₂PO₄)₂ | 2/7 |
| Ca(H₂PO₄)₂.H₂O | 2/7 |
| CaHPO₄ | 1 |
| CaHPO₄.2H₂O | 1 |
| Amorphous Ca₃(PO₄)₂ | 1/2 |
| α-Ca₃(PO₄)₂ | 1/2 |
| β-Ca₃(PO₄)₂ | 1/2 |
| Ca₈H₂(PO₄)₆.5H₂O | 1/3 |

In an alternative preferred embodiment, the first calcium salt is a basic salt selected from calcium oxide, calcium hydroxide and calcium carbonate and the second calcium salt is selected from the salts listed in Table II. The molar quantity of the further calcium salt per mole of the basic salt required to give a total of calcium to phosphate in the preferred ratio of 1.67 : 1 is given in the right-hand column.

**TABLE II**

| Further calcium salt | Mols [per mole of basic salt] |
|---|---|
| Ca(H₂PO₄)₂.H₂O | 3/7 |
| Ca(H₂PO₄)₂ | 3/7 |
| CaHPO₄ | 3/2 |
| CaHPO₄.2H₂O | 3/2 |
| Ca₈H₂(PO₄)₆.5H₂O | 1/2 |
| Amorphous Ca₃(PO₄)₂ | 1/3 |
| α-Ca₃(PO₄)₃ | 1/3 |
| β-Ca₃(PO₄)₃ | 1/3 |

The present invention also provides a kit comprising a liquid and calcium salts as defined above.

The present invention further provides a preparation comprising calcium salts as defined above. It will be appreciated that such a preparation should preferably be desiccated to enhance shelf-life and to prevent premature reaction, especially if the salts are present in a pre-mix.

The invention will be further illustrated with reference to the accompanying Examples.

### Examples

### Comparative Example

A mixture of powders consisting of 72.9 wt% tetracalcium phosphate (mean particle diameter 11µm) and 26.1 wt% anhydrous dicalcium phosphate (mean particle diameter 2.3µm) was prepared, the powder being thoroughly mixed in a blender. 3.5 parts by weight of the mixture of powders and 1 part by weight of 20mM phosphoric acid were then mixed for 30 to 45 seconds and allowed to set. A hydroxyapatite cement was formed. The sample set hard in 38 minutes. The process was repeated for a total of five samples and the diametral tensile strength after setting of each sample was measured (after cement samples had been soaked in water for 24 hours). The mean diametral tensile strength of the five samples was 8.3 ± 1.1MPa. X-ray diffraction topograms of the cement taken at various times after mixing the mixture of powders and the liquid showed that conversion to hydroxyapatite cement went to completion in 4 hours.

### Example 1

3.5 parts by weight of a mixture of powders as described in the Comparative Example were mixed with 1 part by weight of a 0.5 M Na₂HPO₄ solution. The sample set hard in 14 minutes. The process was repeated for five samples and the diametral tensile strength after setting of each sample was measured. The mean diametral strength of the five samples was 10.7 ± 0.8MPa. X-ray diffraction topograms taken at various times after mixing the mixture of powders and the liquid showed that conversion to hydroxyapatite cement went to completion in 2 hours.

Thus it can be seen that the use of a phosphate salt solution has the advantage of shortening the setting time and increasing the strength of the resulting cement.

### Example 2

A mixture of powders consisting of 92.6 wt% β-tricalcium phosphate (mean particle diameter 10µm) and 7.4% wt% CaCO₃ (mean particle diameter 1.8µm) was prepared, the powder being thoroughly mixed in a blender. 4 parts by weight of the mixture of powders and 1 part by weight of 1 M citric acid were mixed and then placed on a glass slab for 30 seconds. The paste hardened in 2.5 minutes. The process was repeated for five samples and the diametral tensile strength after setting of each sample was measured. The mean diametral tensile strength of the five samples was 6.6 ± 0.7MPa.

Thus it can be seen that the use of an organic acid solution permits extremely rapid hardening useful, for example, in the preparation of dental fillings.

## Claims

1. A process for producing a hydroxyapatite cement for use in bone or tooth replacement, comprising admixing a first calcium salt and at least one further calcium salt containing phosphate ions in an aqueous environment to deposit a hydroxyapatite cement, the ratio of the total molar quantity of calcium to the total molar quantity of phosphate being in the range 1.3 : 1 to 1.8 : 1.

2. A process according to Claim 1, wherein the aqueous environment is water.

3. A process for producing a hydroxyapatite cement useful in bone or tooth replacement, comprising admixing a first calcium salt and at least one further calcium salt containing phosphate ions in an aqueous environment so as to deposit a hydroxyapatite cement, characterised in that the aqueous environment essentially consists of an aqueous solution of base, an aqueous solution of a phosphate and/or an aqueous solution of an organic acid capable of forming calcium ligand complexes.

4. A process according to Claim 3, wherein the total concentration of base, phosphate and/or organic acid in solution is at least 0.2 M.

5. A process according to any of Claims 1 to 4, wherein the first calcium salt is Ca₄(PO₄)₂O.

6. A process according to Claim 3 or 4, wherein the first calcium salt is CaO, Ca(OH)₂ and/or CaCO₃.

7. A process according to any preceding Claim, wherein the further calcium salt is selected from Ca(H₂PO₄)₂, Ca(H₂PO₄)₂.H₂O, CaHPO₄, CaHPO₄.2H₂O, amorphous Ca₃(PO₄)₂, α-Ca₃(PO₄)₂, β-Ca₃(PO₄)₂ and/or Ca₈H₂ (PO₄)₆.5H₂O.

8. A process according to any preceding Claim, wherein the ratio of the total molar quantity of calcium to the total molar quantity of phosphate is in the range of 1.3 : 1 to 1.8 : 1, especially substantially in the range 1.6 :1 to 1.75 : 1.

9. A process according to Claim 8, therein said ratio is substantially equal to 1.67 : 1.

10. A process according to any preceding Claim, wherein the aqueous environment comprises an aqueous solution of NaOH and/or KOH.

11. A process according to any preceding Claim, wherein the aqueous environment comprises an aqueous solution of Na₃PO₄, Na₂HPO₄, NaH₂PO₄ and/or (NH₄)₂HPO₄.

12. A process according to any preceding Claim, wherein the aqueous environment comprises an aqueous solution of citric and/or phthalic acid.

13. A preparation for use in a process according to any preceding Claim and comprising, separately or together, a first calcium salt and at least one further calcium salt containing phosphate ions, said salts being sufficently finely divided to react together in an aqueous environment to deposit hydroxyapatite cement, said salts being provided as characterised in any of Claims 1 to 9.

14. A kit for producing a hydroxyapatite cement useful in bone and tooth replacement, comprising a liquid as defined in any of Claims 1 to 12, and, separately, a first calcium salt and at least one further calcium salt containing phosphate ions, said salts being optionally combined and sufficiently finely divided to react together in an aqueous environment to deposit hydroxyapatite cement.

15. A kit according to Claim 14, wherein the first calcium salt and the further calcium salt are provided as characterised in any of Claims 1 to 9.
